# EUROPEAN PATENT APPLICATION

(11) **EP 4 552 655 A1**
(43) Date of publication of application: **14.05.2025**
(21) Application number: 24208841.7
(22) Date of filing: 25.10.2024
(51) Int. Cl.: A61K 47/68, A61P 35/00, C07K 16/30

(54) **ANTIBODY-DRUG CONJUGATES INVOLVING ERIANIN AND THE FABRICATION METHODS AND APPLICATIONS THEREOF**

(30) Priority: 10.11.2023 US 202363598041 P
(71) Applicant: Laboratory for Synthetic Chemistry and Chemical Biology Limited, New Territories Hong Kong (HK)
(72) Inventor: CHE, Chi Ming, Hong Kong (HK); CHU, Chun Ho, Hong Kong (HK)
(74) Representative: Dr. Gassner & Partner mbB

(57) **Abstract**

An antibody-drug conjugate (ADC) including a tumor-targeting antibody covalently bound to at least one erianin-loaded linker is presented. The erianin-loaded linker includes erianin as the cytotoxic agent, an antibody attachment moiety, a cleavable bond and with or without a self-immolative linker. This ADC allows for the targeted delivery of erianin to tumor cells, enhancing therapeutic efficacy while minimizing off-target effects.

## Description

### Cross-Reference to Related Applications:

The present application claims priority from U.S. provisional patent application serial number 63/598,041 filed November 10th, 2023, and the disclosure of which is incorporated herein by reference in its entirety.

### Field of the Invention:

The present invention generally relates to the biotechnology field and pharmaceutical science. More specifically the present invention relates to antibody-drug conjugates involving erianin.

### Background of the Invention:

Cancer, a global health concern, claimed almost 10 million lives in 2020, cementing its position as the leading cause of death. While chemotherapy is a cornerstone of cancer treatment, its widespread use comes with limitations. Many conventional anticancer drugs lack the specificity to selectively target cancer cells, often causing severe systemic toxicity due to their non-selective mode of action. This indiscriminate delivery results in a narrow therapeutic window, leading to undesirable off-target effects and dose-dependent toxicity. To address these issues, there is a pressing need for novel anticancer drugs that exhibit low toxicity toward healthy cells while effectively homing in on tumor sites.

The advent of antibody-drug conjugates (ADCs) has captured significant attention in the realm of anticancer drug research and development. These ADCs comprise monoclonal antibodies that target tumor-specific antigens. These antibodies are then covalently linked to cytotoxic drugs through chemical linkers. This approach capitalizes on the highly specific targeting capabilities of antibodies and the potent cytotoxic properties of these drugs. By combining these attributes, ADCs hold great promise in delivering toxic payloads directly to tumors, thereby widening the therapeutic window.

This promise has been validated by the FDA approval of 14 ADCs, with over 100 others progressing through various stages of clinical trials. However, the current generation of ADCs faces a fundamental challenge. Their payloads are often highly cytotoxic and lack selectivity, affecting both cancer and normal cells. This limitation can lead to significant side effects due to off-target effects in binding to the targeted antigen on non-cancerous cells. Consequently, it is imperative to explore new, safe, and potent payloads to underpin the future development of ADCs, ensuring a more targeted and less toxic approach to cancer treatment.

For thousands of years, Traditional Chinese Medicine (TCM) and natural herbal products have stood the test of time, demonstrating their efficacy in treating various types of cancer. They've gained recognition as a safe alternative anticancer therapy with minimal adverse effects. What sets these remedies apart is their use of natural compounds, like erianin, as opposed to the cytotoxic payloads found in the 14 FDA-approved ADCs.

Erianin, a natural herbal compound, has emerged as a promising anticancer agent. It possesses potent anticancer activity against a spectrum of cancers while maintaining a general safety profile and economic viability. Previous studies have shown that erianin effectively combats various human cancers, including nasopharyngeal carcinoma, hepatocellular carcinoma, oral squamous cell carcinoma, and lung carcinoma. Notably, it achieves this without significantly inhibiting normal cells or causing major organ toxicity.

However, erianin faces challenges that hinder its therapeutic efficiency. It has poor water solubility and undergoes rapid metabolic clearance, leading to low bioavailability. This translates to the need for high effective dosages, increasing the burden on the administered subjects. Addressing these issues can enhance the therapeutic potential of erianin and make it an even more valuable tool in the fight against cancer.

Therefore, the present invention addresses this need.

### Summary of the Invention:

It is an objective of the present invention to provide a compound, composition, or method to solve the aforementioned technical problems.

In accordance with a first aspect of the present invention, an antibody-drug conjugate (ADC) is provided. Specifically, the ADC includes:
a tumor-targeting antibody; and
at least one erianin-loaded linker.

In accordance with one embodiment of the present invention, the tumor-targeting antibody and the at least one erianin-loaded linker are bounded by covalent bonds; and the erianin-loaded linker includes erianin, an antibody attachment moiety, a cleavable bond and with or without a self-immolative linker.

In accordance with one embodiment of the present invention, the tumor-targeting antibody includes one or more thiol groups or one or more amino groups.

In accordance with another embodiment of the present invention, the antibody attachment moiety includes a thiol-reactive Michael acceptor group or an amine-reactive N-hydroxysuccinimide group.

In accordance with another embodiment of the present invention, the tumor-targeting antibody is conjugated with the payload using a non-cleavable linker without the self-immolative linker and cleavable bond.

In accordance with one embodiment of the present invention, at least one of the thiol groups undergoes a Michael addition with the Michael acceptor group of the erianin-loaded linker to conjugate the erianin-loaded linker to the tumor-targeting antibody.

In accordance with another embodiment of the present invention, the at least one of the amino groups undergoes a condensation reaction with the N-hydroxysuccinimide of the erianin-loaded linker to conjugate the erianin-loaded linker to the tumor-targeting antibody.

In accordance with one embodiment of the present invention, the at least one erianin-loaded linker has the following formula: 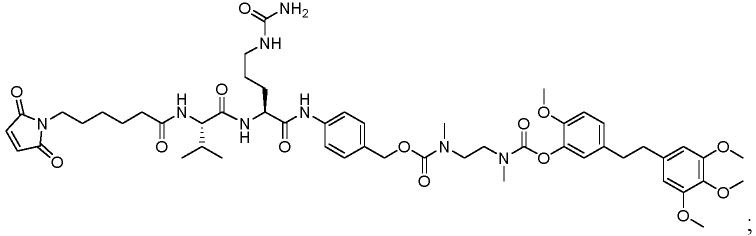 in which the antibody attachment moiety is a maleimide moiety, the cleavable bond is a capthesin B-cleavable Val-Cit unit, and the self-immolative linker is a self-immolative p-aminobenzyl and dicarbamate linker.

In accordance with another embodiment of the present invention, the cleavable bond is any enzymatically cleavable unit, any acid-cleavable unit, or any glutathione-cleavable unit.

In accordance with one embodiment of the present invention, the ADC has 1 to 8 of the erianin-loaded linkers.

In accordance with one embodiment of the present invention, the tumor-targeting antibody targets a tumor-associated receptor selected from HER2, TROP2, Nectin4, folate receptor alpha, EGFR, HER3, or c-MET.

In accordance with one embodiment of the present invention, the tumor-targeting antibody is Trastuzumab.

In accordance with a second aspect of the present invention, a method of fabricating the aforementioned ADC is introduced. The method includes:
synthesizing the erianin-loaded linker; and
conjugating the erianin-loaded linker to the tumor-targeting antibody.

In accordance with one embodiment of the present invention, the method further includes subjecting the tumor-targeting antibody to a reducing treatment for reducing the interchain disulfide bonds to form free thiols on the tumor-targeting antibody before the conjugation.

In accordance with another embodiment of the present invention, the tumor-targeting antibody includes one or more thiol groups or one or more amino groups.

In accordance with one embodiment of the present invention, the antibody attachment moiety includes a thiol-reactive Michael acceptor group or an amine-reactive N-Hydroxysuccinimide group.

In accordance with one embodiment of the present invention, at least one of the thiol groups undergoes a Michael addition with the Michael acceptor group of the erianin-loaded linker to conjugate the erianin-loaded linker to the tumor-targeting antibody.

In accordance with another embodiment of the present invention, the at least one of the amino groups undergoes a condensation reaction with the N-hydroxysuccinimide of the erianin-loaded linker to conjugate the erianin-loaded linker to the tumor-targeting antibody.

In accordance with a third aspect of the present invention, a method of treating a cancer in a subject in need thereof is presented. Particularly, the method includes:
administering a pharmaceutically effective amount of the aforementioned ADC to the subj ect.

In accordance with one embodiment of the present invention, the cancer comprises a breast cancer, lung cancer, colorectal cancer, prostate cancer, pancreatic cancer, liver cancer, esophageal cancer, gastric cancer, ovarian cancer, cervical cancer, endometrial cancer, renal cancer, bladder cancer, thyroid cancer, melanoma, non-Hodgkin lymphoma, Hodgkin lymphoma, multiple myeloma, leukemia, glioblastoma, astrocytoma, medulloblastoma, meningioma, sarcoma, bone cancer, head and neck cancer, testicular cancer, oral cancer, anal cancer, mesothelioma, neuroblastoma, retinoblastoma, and cholangiocarcinoma.

In accordance with a fourth aspect of the present invention, a pharmaceutical composition for treating cancer in a subject in need is provided, Specifically, the composition includes the aforementioned ADC and a pharmaceutically acceptable addition.

In accordance with one embodiment of the present invention, the pharmaceutically acceptable addition comprises an excipient, a stability additive, a carrier, a diluent, and a solubilizer.

In accordance with one embodiment of the present invention, the cancer comprises a breast cancer, lung cancer, colorectal cancer, prostate cancer, pancreatic cancer, liver cancer, esophageal cancer, gastric cancer, ovarian cancer, cervical cancer, endometrial cancer, renal cancer, bladder cancer, thyroid cancer, melanoma, non-Hodgkin lymphoma, Hodgkin lymphoma, multiple myeloma, leukemia, glioblastoma, astrocytoma, medulloblastoma, meningioma, sarcoma, bone cancer, head and neck cancer, testicular cancer, oral cancer, anal cancer, mesothelioma, neuroblastoma, retinoblastoma, and cholangiocarcinoma.

### Brief Description of the Drawings:

The patent or application file contains at least one drawing executed in color. Copies of this patent or patent application publication with color drawing(s) will be provided by the Office upon request and payment of the necessary fee.

Embodiments of the invention are described in more details hereinafter with reference to the drawings, in which:
FIG. 1 depicts the structure of a trastuzumab-erianin ADC according to one embodiment of the present invention;
FIG. 2 depicts the synthetic route of the erianin precursor and the linker-erianin;
FIG. 3 depicts the preparation of trastuzumab-erianin ADC according to one embodiment of the present invention;

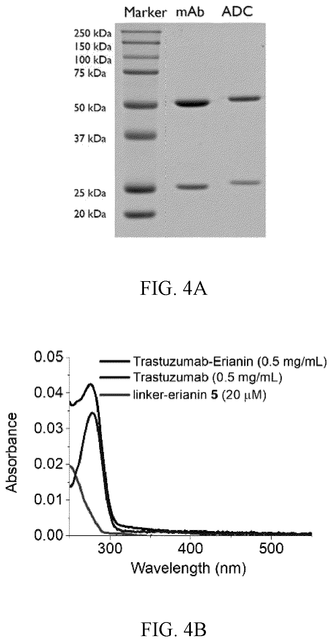

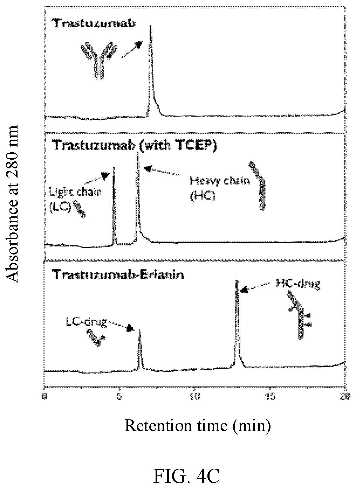

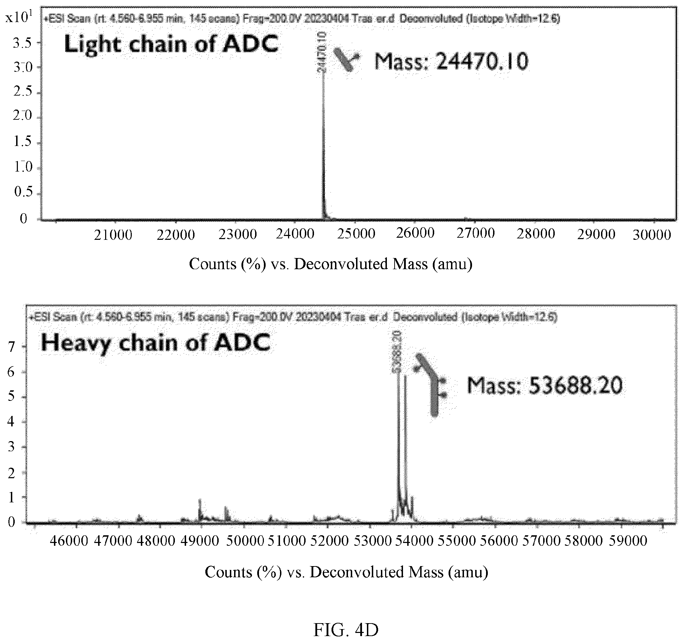

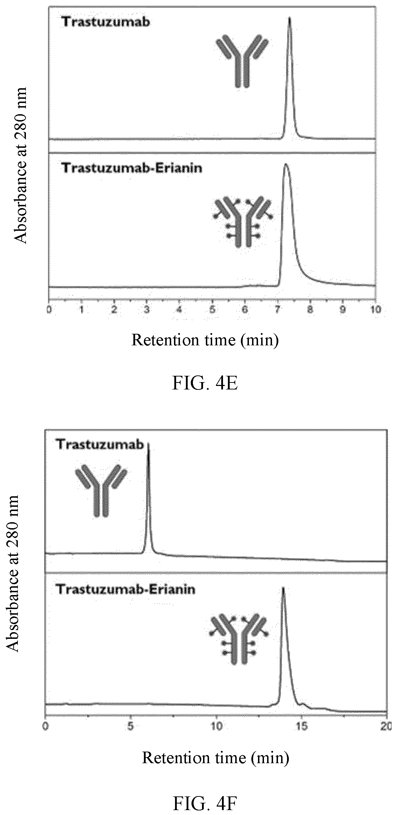

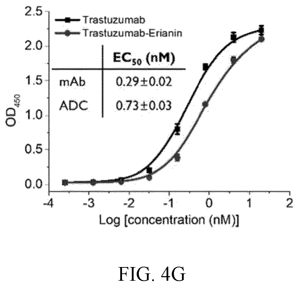

FIGs. 4A-4G depict the characteristics of the fabricated trastuzumab-erianin ADC in accordance of one embodiment of the present invention, in which FIG. 4A shows the reducing SDS-PAGE analysis of trastuzumab (mAb) and trastuzumab-erianin (ADC), FIG. 4B depicts the UV-Vis absorption spectra of ADC, mAb and linker-erianin 5, FIG. 4C shows the RP-HPLC chromatograms of trastuzumab (with or without TCEP reduction) and ADC, FIG. 4D depicts the deconvoluted mass spectra of light chain and heavy chain fragments of ADC, FIG. 4E displays the SEC chromatograms of trastuzumab and ADC, FIG. 4F shows the HIC chromatograms of trastuzumab and ADC, and FIG. 4G depicts the binding of mAb and ADC to HER2 protein by ELISA;

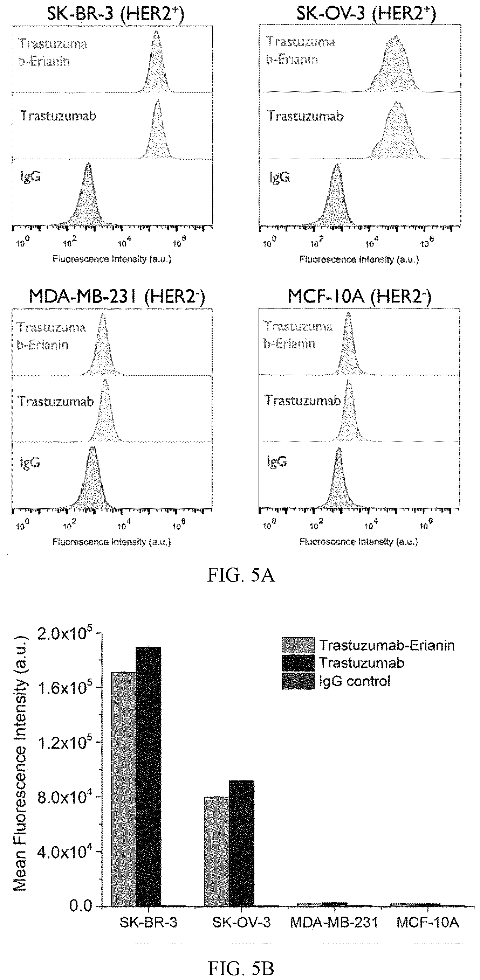

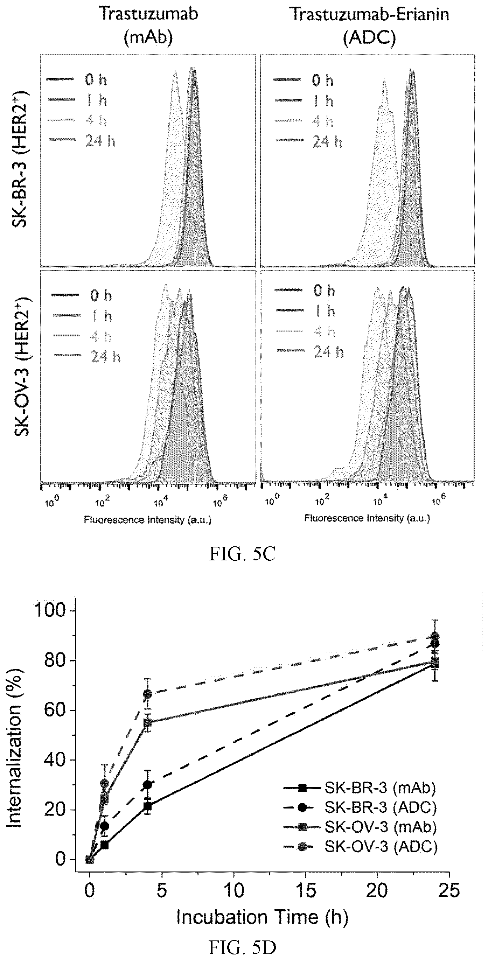

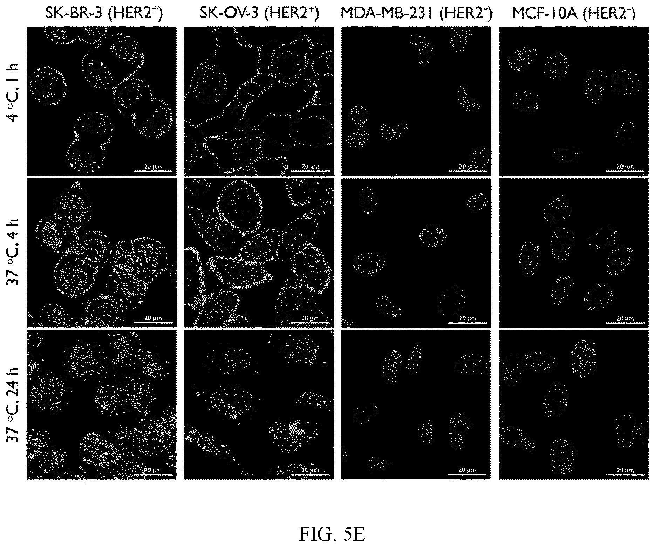

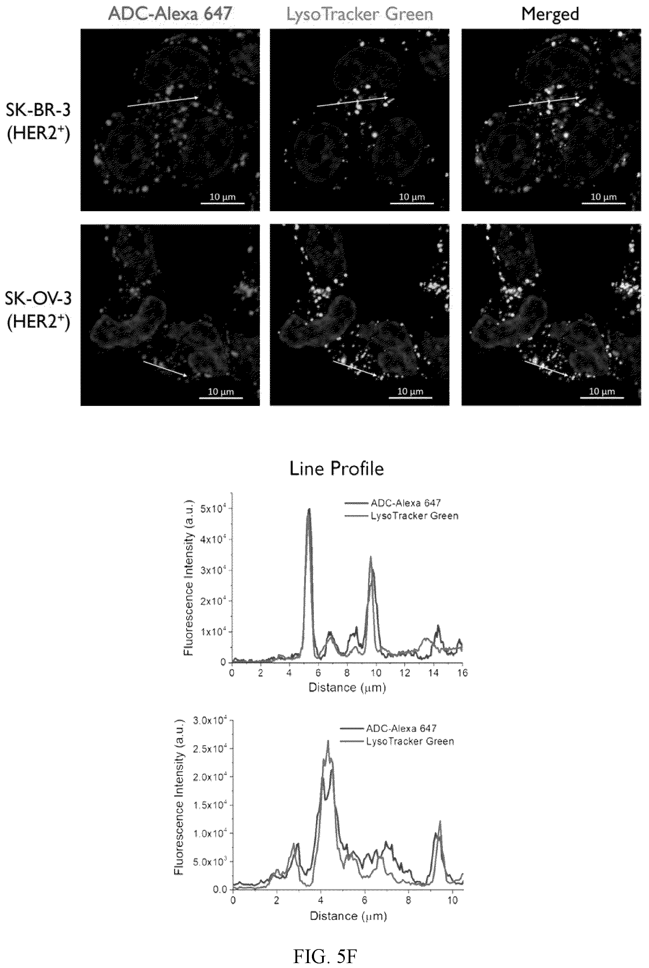

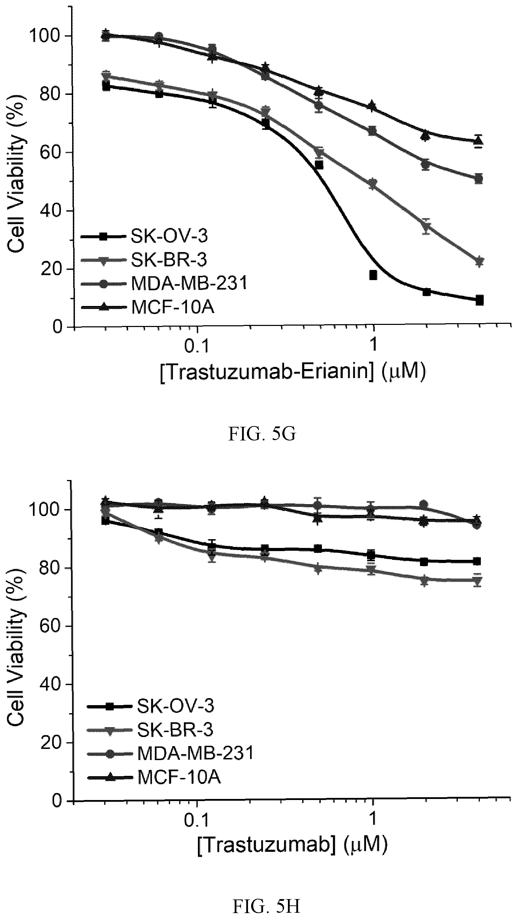

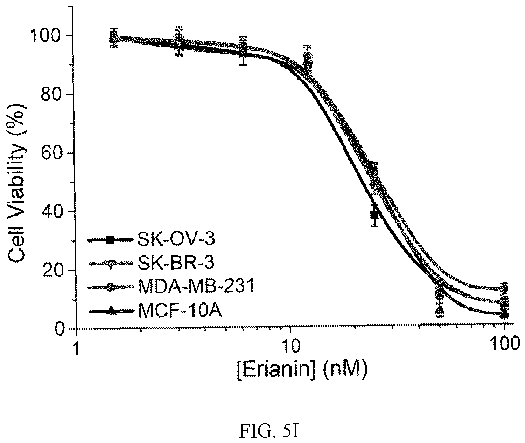

FIGs. 5A-5I depict the *in vitro* study of trastuzumab-erianin ADC, in which FIG. 5A shows the binding activity analysis of ADC, mAb and IgG control and FIG. 5B depicts the corresponding quantified flow cytometric data, FIG. 5C depicts the internalization analysis of ADC and mAb and FIG. 5D displays the corresponding quantified flow cytometric data, FIG. 5E demonstrates the confocal microscopic images of ADC in different cell lines, FIG. 5F shows the colocalization of ADC with LysoTracker Green, FIG. 5G depicts the *in vitro* anti-proliferative study of ADC, FIG. 5H shows the *in vitro* anti-proliferative study of trastuzumab, and FIG. 5I depicts the *in vitro* anti-proliferative study of erianin; and
FIGs. 6A-6E depict the *in vivo* study of trastuzumab-erianin ADC, in which FIG. 6A is a schematic representation of the *in vivo* experiment, FIG. 6B depicts the tumor growth curves of SK-OV-3 tumor-bearing mice being treated with erianin, trastuzumab and ADC and the vehicle control, FIG. 6C displays the image of the tumor after 28 days of treatment, FIG. 6D depicts the tumor weight of the mice after 28 days of treatment, and FIG. 6E shows the body weight of the mice during 28 days.

### Detailed Description:

In the following description, compounds, compositions, and/or methods of antibody-drug conjugate involving erianin and the likes are set forth as preferred examples. It will be apparent to those skilled in the art that modifications, including additions and/or substitutions may be made without departing from the scope and spirit of the invention. Specific details may be omitted so as not to obscure the invention; however, the disclosure is written to enable one skilled in the art to practice the teachings herein without undue experimentation.

The term "antibody-drug conjugate (ADC)" refers to a targeted cancer therapy that combines an antibody specific to cancer cells with a cytotoxic drug (also known as a payload). The antibody is designed to recognize and bind to specific antigens present on the surface of cancer cells, while the cytotoxic drug is responsible for killing the cancer cells. Once the ADC binds to its target antigen, it is internalized into the cancer cell, where the cytotoxic drug is released, leading to cell death. ADCs are designed to specifically target cancer cells, thereby reducing the damage to normal, healthy cells and minimizing side effects compared to traditional chemotherapy.

In accordance with a first aspect of the present invention, an ADC is provided. The ADC aims to deliver a potent anti-tumor agent, erianin, directly to tumor cells, thereby minimizing systemic toxicity and enhancing therapeutic efficacy. The ADC includes a tumor-targeting antibody, which is conjugated to one or more erianin-loaded linkers. These linkers play a critical role in the ADC's functionality, as they serve to attach erianin to the antibody and release it within the tumor environment.

The erianin-loaded linker is a multi-component structure consisting of erianin itself, an antibody attachment moiety, a cleavable bond and with or without a self-immolative linker. The cleavable bond is designed to be sensitive to tumor-specific conditions, allowing for targeted release of erianin within the tumor site. This specificity is further enhanced by the tumor-targeting antibody, which selectively binds to receptors that are overexpressed on the surface of tumor cells.

As used herein, the term "cleavable bond" and "self-immolative linker" relates to chemical linkers that connect the cytotoxic drug (payload) to the monoclonal antibody. Upon reaching the target cancer cell, these linkers are designed to undergo a triggered breakdown or "self-immolation," resulting in the release of the active drug in its active form. When the antibody-drug conjugate is administered into the bloodstream, the monoclonal antibody component binds to the specific antigen on the surface of a cancer cell. The antigen is typically a protein that is overexpressed on the cancer cell but not on normal cells.

Once the antibody-drug conjugate binds to its target antigen, the complex is internalized by the cancer cell through endocytosis. Inside the cell, the antibody-drug conjugate is transported to the lysosome, a cellular organelle that contains enzymes responsible for breaking down proteins and other molecules. In the lysosome or other specific cellular environments, the cleavable bond is cleaved in response to specific stimuli. These stimuli can include:
(a) Enzymatic Cleavage: Specific enzymes present in the lysosome, such as cathepsins, can cleave the linker.
(b) Reduction: Some linkers (e.g., disulfide bond) respond to the reductive environment inside the cell (e.g., presence of glutathione).
(c) Acidic pH: The acidic environment of the lysosome can also trigger cleavage.

After the initial cleavage, the self-immolative linker undergoes a rapid and spontaneous breakdown. This breakdown is often a series of intramolecular reactions that result in the complete dissociation of the linker, thereby releasing the cytotoxic drug in its active form.

Once the cytotoxic drug is released, it can interact with its intracellular targets (e.g., DNA, tubulin), leading to cell death. The design ensures that the drug is only released within the target cancer cell, minimizing damage to healthy tissues.

The tumor-targeting antibody used in this ADC can include one or more thiol groups or one or more amino groups, providing multiple points of attachment for the erianin-loaded linkers. The antibody attachment moiety is tailored to these functional groups, with options including a thiol-reactive Michael acceptor group or an amine-reactive N-hydroxysuccinimide (NHS) group. When the antibody possesses thiol groups, these can undergo a Michael addition reaction with the Michael acceptor group of the erianin-loaded linker, effectively attaching the erianin to the antibody. Alternatively, if the antibody contains amino groups, these can participate in a condensation reaction with the N-hydroxysuccinimide group of the linker, facilitating the attachment of the erianin-loaded linker to the antibody.

In one embodiment of the invention, the erianin-loaded linker has the following formula:

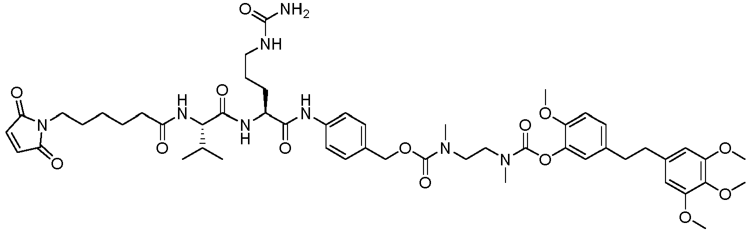

The erianin-loaded linker has a specific chemical structure. The antibody attachment moiety is a maleimide moiety, chosen for its ability to react with thiol groups on the antibody. The cleavable bond within the linker is a cathepsin B-cleavable Val-Cit unit, which is stable in the bloodstream but is cleaved in the lysosomal environment of tumor cells. This cleavage triggers the self-immolative linker, which includes a p-aminobenzyl and dicarbamate linker, to release erianin within the tumor cell, thereby exerting its cytotoxic effects.

The ADC is designed to carry between 1 and 8 erianin-loaded linkers, allowing for flexibility in drug loading depending on the therapeutic needs. The tumor-targeting antibody may be specific for a variety of tumor-associated receptors, including but not limited to HER2, TROP2, Nectin4, folate receptor alpha, EGFR, HER3, or c-MET, making the ADC applicable to a wide range of cancer types. In a preferred embodiment, the tumor-targeting antibody is Trastuzumab, a well-known monoclonal antibody that targets the HER2 receptor, commonly overexpressed in certain types of breast cancer.

In some embodiments, the tumor-associated receptors include, but not limited to, PD-L1, VEGF, CD20, CD19, CD22, CD33, PSMA, MUC1, CD30, CD38, mesothelin, EpCAM, c-MET, RANKL, glypican-3 and CD47.

In addition to trastuzumab, several other antibodies may be used in antibody-drug conjugates of the present invention. Examples of such antibodies and their target antigens are listed below:
Brentuximab Vedotin (Adcetris):
   Antibody: Brentuximab (anti-CD30).
   Target Antigen: CD30.
   Indications: Used primarily for Hodgkin lymphoma and anaplastic large cell lymphoma.
Inotuzumab Ozogamicin (Besponsa):
   Antibody: Inotuzumab (anti-CD22).
   Target Antigen: CD22.
   Indications: Treats relapsed or refractory B-cell precursor acute lymphoblastic
   leukemia (ALL).
Loncastuximab Tesirine (Zynlonta):
   Antibody: Loncastuximab (anti-CD19).
   Target Antigen: CD19.
   Indications: Large B-cell lymphoma
Sacituzumab Govitecan (Trodelvy):
   Antibody: Sacituzumab (anti-TROP-2).
   Target Antigen: TROP-2.
   Indications: Metastatic triple-negative breast cancer and urothelial cancer.
Gemtuzumab Ozogamicin (Mylotarg):
   Antibody: Gemtuzumab (anti-CD33).
   Target Antigen: CD33.
   Indications: Acute myeloid leukemia (AML).
Enfortumab Vedotin (Padcev):
   Antibody: Enfortumab (anti-Nectin-4).
   Target Antigen: Nectin-4.
   Indications: Locally advanced or metastatic urothelial carcinoma.
Polatuzumab Vedotin (Polivy):
   Antibody: Polatuzumab (anti-CD79b).
   Target Antigen: CD79b.
   Indications: Diffuse large B-cell lymphoma (DLBCL).
Tisotumab Vedotin (Tivdak):
   Antibody: Tisotumab (anti-Tissue Factor, TF).
   Target Antigen: Tissue Factor (TF).
   Indications: Cervical cancer and solid tumors, including liver cancer.
Mirvetuximab Soravtansine (Elahere):
   Antibody: Mirvetuximab (anti-Folate Receptor, FRα).
   Target Antigen: FRα.
   Indications: Ovarian, fallopian tube and peritoneal cancers
Glypican-3 (GPC3)-Targeted ADCs:
   Antibody: Experimental antibodies targeting GPC3.
   Target Antigen: Glypican-3 (GPC3).
   Indications: GPC3 is a cell surface protein commonly overexpressed in liver cancer, making it a promising target for ADC development.
Cetuximab Sarotalocan :
   Antibody: Cetuximab (anti-EGFR).
   Target Antigen: Epidermal Growth Factor Receptor (EGFR).
   Indications: Unresectable locally advanced and recurrent head and neck cancer.
Nimotuzumab:
   Antibody: Nimotuzumab (anti-EGFR).
   Target Antigen: EGFR.
   Indications: Nasopharyngeal carcinoma (NPC) and other head and neck cancers, especially in regions with a high incidence of NPC.
Datopotamab Deruxtecan (Dato-DXd):
   Antibody: Datopotamab (anti-TROP-2).
   Target Antigen: TROP-2.
   Indications: Under investigation for non-small cell lung cancer (NSCLC) and other solid tumors.
Amivantamab (Rybrevant):
   Antibody: Amivantamab (bi-specific anti-EGFR and anti-MET).
   Target Antigen: EGFR and MET.
   Indications: Approved for NSCLC with EGFR exon 20 insertion mutations, combining EGFR and MET targeting.
Zanidatamab (ZW25):
   Antibody: Zanidatamab (bi-specific anti-HER2).
   Target Antigen: HER2.
   Indications: Investigated in a variety of HER2-expressing cancers, including gastroesophageal, breast, and potentially lung cancers.
Patritumab Deruxtecan:
   Antibody: Patritumab (anti-HER3).
   Target Antigen: HER3.
   Indications: Locally advanced or metastatic EGFR-mutated non-small cell lung cancer.
Telisotuzumab Vedotin:
   Antibody: Telisotuzumab (anti-c-MET).
   Target Antigen: c-MET.
   Indications: Advanced/metastatic EGFR wild-type, nonsquamous NSCLC with high levels of c-Met overexpression.

In some embodiments, the cleavable bond is selected from the group consisting of a protease-sensitive peptide linker, a disulfide bond, and an acid-labile bond; and the self-immolative linker is selected from the group consisting of p-aminobenzyl alcohol, p-hydroxybenzyl alcohol, and disulfide-based self-immolative linkers.

This innovative ADC leverages the specificity of tumor-targeting antibodies combined with the potent anti-tumor activity of erianin, delivered in a controlled and targeted manner via a sophisticated linker system, to provide a powerful and effective treatment option for cancer patients.

In accordance with a second aspect of the present invention, a method for fabricating the aforementioned ADC is introduced. The method involves several critical steps that ensure the successful synthesis and conjugation of the erianin-loaded linker to the tumor-targeting antibody. Initially, the process begins with the synthesis of the erianin-loaded linker. This involves chemically loading the cytotoxic agent, erianin, onto a suitable linker molecule that is designed to facilitate the attachment to the antibody. The selection of the linker is crucial, as it must possess the necessary functional groups to enable subsequent conjugation to the antibody while maintaining the bioactivity of erianin.

Once the erianin-loaded linker has been synthesized, the next step involves conjugating this linker to the tumor-targeting antibody. This process requires precise handling and preparation of the antibody to ensure effective conjugation. To optimize the antibody for conjugation, it may be subjected to a reducing treatment. This treatment is designed to reduce the interchain disulfide bonds within the antibody, thereby forming free thiol groups on the antibody. These free thiol groups are essential for the subsequent conjugation process. For instance, the reducing treatment includes exposing the antibody to a reducing agent, such as tris(2-carboxyethyl) phosphine hydrochloride to produce free thiols on the antibody by reduction of interchain disulfide bonds present in the antibody.

The tumor-targeting antibody itself may naturally contain one or more thiol groups or one or more amino groups, both of which are potential sites for conjugation. The choice of conjugation strategy depends on the specific functional groups present on the antibody. For instance, if the antibody contains thiol groups, the conjugation can be achieved through a reaction with a thiol-reactive Michael acceptor group present on the erianin-loaded linker. This reaction, known as Michael addition, facilitates the formation of a stable bond between the thiol group on the antibody and the Michael acceptor group on the linker, effectively attaching the erianin payload to the antibody.

Alternatively, if the antibody contains amino groups, the conjugation process can be carried out using an amine-reactive NHS group present on the erianin-loaded linker. In this case, the conjugation involves a condensation reaction between the amino group on the antibody and the NHS group on the linker. This reaction results in the formation of a stable amide bond, securely attaching the erianin-loaded linker to the antibody.

These steps culminate in the formation of the final ADC, where the erianin drug is effectively linked to the tumor-targeting antibody via the synthesized linker. The resulting ADC is designed to target and deliver the therapeutic erianin specifically to tumor cells, maximizing therapeutic efficacy while minimizing off-target effects.

This method provides a robust and versatile approach for fabricating ADCs, allowing for the customization of the conjugation process based on the specific functional groups present on the tumor-targeting antibody. By carefully selecting and synthesizing the appropriate erianin-loaded linker and optimizing the conjugation conditions, the method ensures the production of highly effective and targeted ADCs for cancer therapy.

In accordance with a third aspect of the present invention, a method for treating cancer in a subject in need thereof through the administration of a pharmaceutically effective amount of the aforementioned ADC.

The ADC is fabricated to selectively target tumor cells, minimizing damage to healthy tissues and reducing the adverse side effects typically associated with traditional chemotherapy. By delivering a potent cytotoxic agent, such as erianin, directly to the cancer cells via a tumor-targeting antibody, the ADC enhances the therapeutic efficacy while ensuring that the drug is concentrated where it is most needed-within the tumor microenvironment.

The method of treatment is applicable to a wide range of cancers, making it a versatile and valuable tool in oncology. The cancers that can be treated using this method include, but are not limited to, breast cancer, lung cancer, colorectal cancer, prostate cancer, pancreatic cancer, liver cancer, esophageal cancer, gastric cancer, ovarian cancer, cervical cancer, and endometrial cancer. Additionally, this method is effective against renal cancer, bladder cancer, thyroid cancer, melanoma, non-Hodgkin lymphoma, Hodgkin lymphoma, multiple myeloma, and various types of leukemia, such as glioblastoma, astrocytoma, medulloblastoma, and meningioma. Furthermore, the method extends its therapeutic reach to sarcomas, bone cancers, and cancers of the head and neck, as well as testicular cancer, oral cancer, and anal cancer. It also shows promise in treating mesothelioma, neuroblastoma, retinoblastoma, and cholangiocarcinoma. The wide spectrum of cancers addressed by this method underscores its potential as a comprehensive treatment option in the field of oncology.

In practice, the method involves administering the ADC in a dose that is pharmaceutically effective, meaning it is sufficient to achieve the desired therapeutic outcome, which is the reduction or elimination of cancer cells in the subject. The specific dosage and administration schedule would be tailored to the individual patient's condition, taking into account factors such as the type and stage of cancer, the patient's overall health, and the presence of any other medical conditions.

In accordance with a fourth aspect of the present invention, a pharmaceutical composition specifically manufactured for treating cancer in a subject in need. This composition incorporates the aforementioned ADC, which is combined with a pharmaceutically acceptable addition to form a complete therapeutic solution. The pharmaceutically acceptable addition is not merely a filler but is critical to the functionality and stability of the ADC within the composition. It may include various components such as excipients, stability additives, carriers, diluents, and solubilizers. Each of these components plays a crucial role in ensuring the proper delivery and efficacy of the ADC, as well as maintaining the stability of the formulation over time.

Furthermore, the pharmaceutical composition is applicable to a wide range of cancers, demonstrating its versatility and potential for broad therapeutic use. The cancers targeted by this composition include, but are not limited to, breast cancer, lung cancer, colorectal cancer, prostate cancer, pancreatic cancer, liver cancer, esophageal cancer, gastric cancer, ovarian cancer, cervical cancer, endometrial cancer, renal cancer, bladder cancer, thyroid cancer, melanoma, non-Hodgkin lymphoma, Hodgkin lymphoma, multiple myeloma, leukemia, glioblastoma, astrocytoma, medulloblastoma, meningioma, sarcoma, bone cancer, head and neck cancer, testicular cancer, oral cancer, anal cancer, mesothelioma, neuroblastoma, retinoblastoma, and cholangiocarcinoma. This extensive range underscores the composition's potential utility across numerous cancer types, providing a robust approach to cancer treatment tailored to the needs of diverse patient populations.

The present invention will be further understood by reference to the following non limiting examples.

### EXAMPLES

### Materials and methods

### Preparation of reactive carbonate 1

A mixture of erianin (150 mg, 0.47 mmol), 4-nitrophenyl chloroformate (190 mg, 0.94 mmol), and pyridine (150 µL, 1.88 mmol) in CH₂Cl₂ (10 mL) is stirred at room temperature for 4 h. The solvent is then evaporated under reduced pressure, and the residue is purified by column chromatography on silica gel with hexane/ethyl acetate (3:1 v/v) as the eluent to afford **1** (220 mg, 96%). ¹H NMR (600 MHz, CDCl₃): δ 8.31 (d, *J* = 9.0 Hz, 2H), 7.49 (d, *J* = 9 Hz, 1H), 7.03-7.04 (m, 2H), 6.93 (d, *J* = 8.4 Hz, 1H), 6.33 (s, 1H), 3.88 (s, 3H), 3.82 (s, 3H), 3.81 (s, 6H), 2.83-2.90 (m, 4H). ¹³C NMR (150 MHz, CDCl₃): δ 155.67, 153.22, 150.75, 149.16, 145.65, 139.56, 137.14, 136.37, 134.61, 127.74, 125.5, 122.16, 121.83, 112.69, 105.54, 61.01, 56.29, 56.18, 38.32, 37.06. HRMS (ESI): m/z for C₂₅H₂₆NO₉⁺ [M+H]⁺ calcd:484.1608; found 484.1617.

### Preparation of compound 2

A mixture of **1** (220 mg, 0.46 mmol), tert-butyl methyl(2-(methylamino)ethyl)carbamate (171 mg, 0.91 mmol) and triethylamine (0.25 mL, 1.82 mmol) in CH₂Cl₂ (10 mL) is stirred at room temperature for overnight. The solvent is then evaporated under reduced pressure, and the residue is purified by column chromatography on silica gel with hexane/ethyl acetate (3:1 v/v) as the eluent to afford **2** (240 mg, 99%). ¹H NMR (600 MHz, CDCl₃): δ 6.84-6.97 (m, 3H), 6.37 (s, 1H), 3.83 (s, 6H), 3.82 (s, 3H), 3.80 (s, 3H), 3.43-3.60 (m, 4H), 3.04-3.14 (m, 3H), 2.94 (s, 3H), 2.83 (s, 4H), 1.46 (s, 9H). ¹³C NMR (150 MHz, CDCl₃): δ 154.60, 153.18, 150.00, 137.66, 136.23, 134.29, 126.26, 123.49, 123.35, 115.70, 112.43, 112.16, 105.46, 61.01, 56.19, 56.03, 38.40, 37.19, 35.64, 34.94, 28.58. HRMS (ESI): m/z for C₂₈H₄₁N₂O₈⁺ [M+H]⁺ calcd:533.2863; found 533.2861.

### Preparation of erianin precursor 3

Compound **2** is dissolved in CH₂Cl₂ (5 mL), followed by the addition of trifluoroacetic acid (2 mL). The mixture is stirred at room temperature for 1 h and then the solvent is removed in vacuo. The crude product **3** is used for the subsequent experiment without purification.

### Preparation of compound 4

A mixture of **3** (200 mg, 0.46 mmol), Fmoc-Val-Cit-PAB-PNP (180 mg, 0.23 mmol) and *N*,*N-*diisopropylethylamine (0.82 mL, 4.69 mmol) in DMF (5 mL) is stirred at room temperature for overnight. The solvent is then evaporated under reduced pressure, and the residue is purified by column chromatography on silica gel with CHCl₃/CH₃OH (50:1 v/v) as the eluent to afford **4** (192 mg, 77%). ¹H NMR (600 MHz, DMSO-d₆): δ 10.08 (s, 1H), 8.14 (d, *J =* 7.2 Hz, 1H), 7.89 (d, *J =* 7.8 Hz, 2H), 7.73-7.76 (m, 2H), 7.33-7.58 (m, 3H) 7.28-7.31 (m, 6H), 6.94-7.03 (m, 3H), 6.48-6.53 (m, 2H), 5.97 (s, 1H), 5.42 (s, 2H), 5.02 (s, 2H), 4.30 (s, 1H), 4.22-4.23 (m, 3H), 3.93 (t, *J* = 7.8 Hz, 1H), 3.69-3.72 (m, 9H), 3.60 (s, 3H), 3.42-3.53 (m, 4H), 2.77-3.02 (m, 12H), 2.00-2.03 (m, 1H), 1.35-1.70 (m, 4H), 0.84-0.88 (m, 6H). ¹³C NMR (150 MHz, DMSO-d₆): δ 171.29, 170.59, 158.90, 156.13, 155.65, 155.49, 155.38, 153.83, 153.64, 152.64, 149.59, 143.92, 143.78, 140.72, 139.87, 139.73, 138.59, 137.24, 137.15, 135.56, 133.91, 133.81, 131.78, 131.55, 128.45, 128.34, 128.25, 127.65, 127.08, 125.98, 125.38, 123.13, 120.09, 119.01, 112.53, 112.42, 112.31, 105.60, 79.19, 65.69, 60.08, 59.94, 55.76, 55.71, 53.10, 46.70, 46.47, 46.29, 46.03, 45.92, 45.52, 38.60, 37.56, 37.45, 36.14, 36.07, 34.74, 34.67, 34.60, 34.53, 34.19, 34.06, 30.47, 29.49, 26.80, 19.23, 1819.28. HRMS (ESI): m/z for C₇₀H₇₁N₇O₁₃C₅₇H₇₁N₇O₁₃⁺ [M+H]⁺ calcd: 1060.5032; found: 1060.5020.

### Preparation of linker-erianin 5

Compound **4** (172 mg, 0.16 mmol) is treated with 20% piperidine in DMF (5 mL) at room temperature for 30 min and then the solvent is removed in vacuo. The intermediate is further reacted with 6-maleimidocaproic acid N-succinimidyl ester (100 mg, 0.32 mmol) in the presence of triethylamine (0.11 mL, 0.81 mmol) in DMF (5 mL) at room temperature for 2 h. The solvent is then evaporated under reduced pressure, and the residue is purified by column chromatography on silica gel with CHCl₃/CH₃OH (20:1 v/v) as the eluent to afford 5 (130 mg, 77%). ¹H NMR (600 MHz, DMSO-d₆): δ 9.99 (s, 1H), 8.09 (d, *J* = 7.2 Hz, 1H), 7.81 (d, *J* = 8.4 Hz, 1H), 7.50-7.58 (m, 2H), 7.21-7.35 (m, 2H), 6.93-7.03 (m, 5H), 6.48-6.52 (m, 2H), 5.97 (s, 1H), 5.41 (s, 2H), 5.01 (s, 2H), 4.36-4.37 (m, 1H), 3.72 (t, *J* = 7.8 Hz, 1H), 3.70-3.73 (m, 9H), 3.60 (s, 3H), 3.41-3.49 (m, 6H), 2.77-3.02 (m, 12H), 2.12-2.16 (m, 2H), 2.00-2.03 (m, 1H), 1.04-1.48 (m, 10H), 0.81-0.85 (m, 6H). ¹³C NMR (150 MHz, DMSO-d₆): δ 172.72, 171.75, 171.54, 171.04, 159.33, 153.09, 150.04, 139.06, 136.01, 134.91, 128.93, 126.42, 123.60, 119.44, 106.06, 79.65, 60.40, 58.01, 56.49, 56.17, 53.56, 37.47, 35.39, 31.17, 30.85, 29.76, 28.23, 27.28, 26.25, 25.69, 19.71, 19.03, 18.65. HRMS (ESI): m/z for C₇₀H₇₁N₇O₁₃C₅₇H₇₁N₇O₁₃⁺ [M+H]⁺ calcd: 1031.5090; found: 1031.5092.

### Preparation and characterization of Trastuzumab-Erianin ADC

Trastuzumab (MCE, 2.5 mg/mL) is dissolved in borate buffer (pH 8.0) and TCEP (10 equiv.) is added to the reaction mixture for 1.5 h. After the reduction of disulphide bond, linker-erianin **5** (12 equiv.) dissolved in DMSO (2% v/v) is added and the reaction mixture is stirred for 2 h. The trastuzumab-erianin ADC is purified by using desalting column (Thermo Fisher, 7K MWCO) according to the manufacturer's protocol. The protein concentration is determined by the standard bicinchoninic acid (BCA) assay and the yield is determined to be 87%. The conjugate is analyzed by Thermo Scientific µDrop and reducing SDS-PAGE with 12% acrylamide with 4% stacking gel as standard.

The antibody and the ADC are analyzed on a MAbPac Reversed Phase HPLC Column (4 µm, 3 mm × 50 mm) at a flow rate of 0.3 mL/min. Electrospray ionization (ESI) mass spectra are recorded on an Agilent 6546 LC/Q-TOF mass spectrometer. The condition used for the analysis is set as follows: solvent A = 0.1% FA in deionized water and solvent B = 0.1% FA in acetonitrile; gradient: 80% A + 20% B in the first 0.5 min, then changed to 40% A + 60% B in 3 min, maintained under this condition for 5 min, further changed to 80% A + 20% B in 0.5 min, maintained under this condition for 1 min.

The average drug-to-antibody (DAR) ratio is determined by using the corresponding peak areas of the chromatograms in the deconvoluted mass spectra. For the size-exclusion chromatography, the antibody and the ADC are analyzed on a Zenix-C SEC-300 Column (3 µm, 7.8 mm × 300 mm) at a flow rate of 1.0 mL/min with an isocratic eluent (150 mM sodium phosphate, pH 7.0).

For the hydrophobic interaction chromatography, the antibody and the ADC are analyzed on a MAbPac^{™} HIC-Butyl HPLC Column (5 µm, 4.6 mm × 100 mm) at a flow rate of 1.0 mL/min. The condition used for the analysis is set as follows: solvent A = 1.5 M ammonium sulfate, 50 mM sodium phosphate, pH 7.0/isopropanol (95:5 v/v) and solvent B = 50 mM sodium phosphate, pH 7.0/isopropanol (80:20 v/v); gradient: 100% A + 0% B in the first 1 min, then changed to 0% A + 100% B in 14 min, maintained under this condition for 5 min, further changed to 100% A + 0% B in 1 min, maintained under this condition for 4 min.

### Flow cytometric study

Different cells (5 × 10⁵ cells/tube) are suspended in FACS staining buffer (2% FBS in PBS) into a 1.5 mL eppendorf tube. They are incubated with trastuzumab, ADC or IgG respectively in staining buffer (100 µL) at 4°C. After 1 h, the cell suspensions are centrifuged at 1000 rpm for 3 min at 4°C and washed with staining buffer (1 mL) twice to discard unbound antibodies. The cells are then incubated with secondary antibody at 4°C for 1 h and then subjected to centrifugation. The cells are washed twice again and resuspended in staining buffer. The mean fluorescence intensities (MFI) are measured using Beckman Cytoflex S Flow Cytometer. For the internalization study, the cells, after being treated with trastuzumab or ADC at 4°C for 1 h, are incubated at 37°C for different periods of time, followed by staining with secondary antibody. The degree of internalization is determined by the percentage of decrease in the MFI of cells incubated at 37°C compared to the control incubated at 4°C. The internalization efficiency is calculated by the equation: internalization efficiency (%) = [(MFI of the cells at 4°C - MFI of the cells incubated at 37°C)/MFI of the cells at 4°C] × 100%.

### Confocal microscopic study

Different cells (1 × 10⁵ cells/tube) are seeded into 35 mm confocal culture dishes. After 24 h of incubation, the media are removed and washed with ice-cold PBS and then placed on ice. The cells are incubated with ADC, which is pre-labelled with Alexa Fluor^{™} 647 NHS Ester (Invitrogen), at 4°C for 1 h. The solutions are then removed, and the cells are rinsed with ice-cold PBS twice before being examined with Zeiss LSM900 Confocal Microscope. To observe the internalization, the cells are incubated at 37°C for 4 h or 24 h. After 24 h, the cells are further stained with LysoTracker Green DND-26 to detect lysosomes.

### Example 1. The preparation of Trastuzumab-Erianin ADC

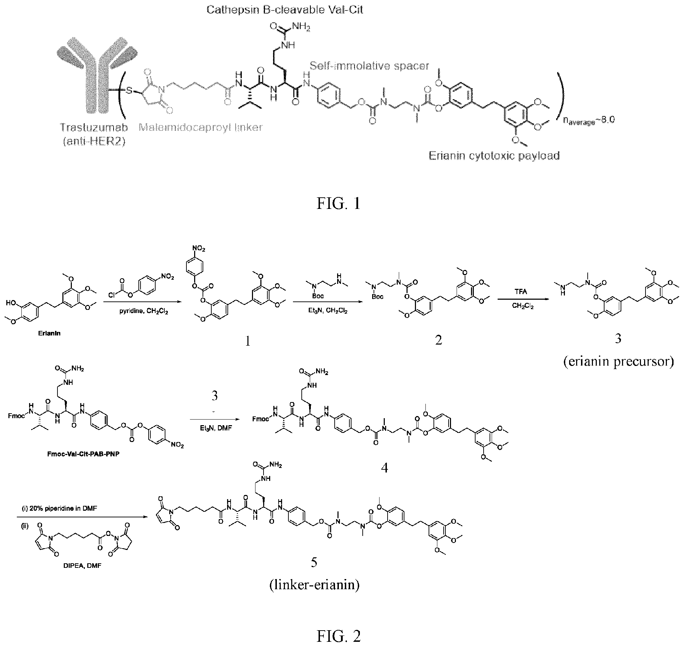

Due to the high HER2-binding affinity and specificity, trastuzumab has been widely studied in ADC development and approved to target several HER2-overexpressed cancers. Currently, Enhertu (fam-trastuzumab deruxtecan-nxki) and Kadcyla (ado-trastuzumab emtansine) are the two FDA-approved HER2-specific ADCs for the treatment of early and metastatic breast cancer. As a proof of concept, trastuzumab is chosen as the tumor-directing antibody and conjugated with erianin through a dipeptide (Val-Cit) cathepsin B cleavable linker and a short self-immolative spacer (FIG. 1). The linker is then connected to a cysteine residue of the antibody by a maleimidocaproyl moiety through the conventional Michael addition after the reduction of the interchain disulfide bonds of the antibody. It is expected that upon binding with the HER2 receptors on cancer cell surface, the ADC might be internalized through receptor-mediated endocytosis into lysosomes and further digested by cathepsin B, leading to the linker cleavage and drug release.

FIG. 2 shows the synthetic route of the erianin precursor and the linker-erianin fragment. Owing to the simple chemical structure, erianin can be easily modified by making use of its phenol group. Firstly, erianin is converted to reactive carbonate **1,** followed by condensation with Boc-protected ethylenediamine spacer to give compound **2.** Subsequent acid deprotection can afford erianin precursor **3,** which is then conjugated with Fmoc-Val-Cit-PAB-PNP to give compound **4.** After the Fmoc deprotection and condensation with 6-maleimidocaproic acid N-succinimidyl ester, the linker-erianin **5** is prepared.

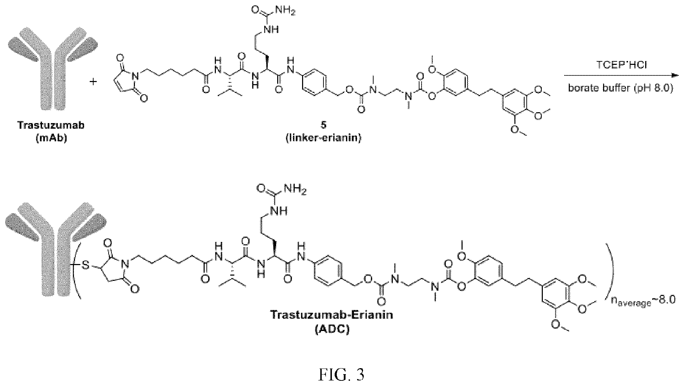

Finally, trastuzumab is conjugated with linker-erianin **5** using the conventional cysteine conjugation strategy (FIG. 3). The four interchain disulfide bonds of the antibody is reduced with tris(2-carboxyethyl) phosphine hydrochloride (TCEP·HCl). The free cysteine thiols are then reacted with **5** in situ through Michael addition. After removal of the unreacted **5** by Zeba spin desalting column (7 kDa MWCO), trastuzumab-erianin ADC is obtained. The protein concentration is determined by the standard bicinchoninic acid (BCA) assay and the yield is determined to be 87%.

### Example 2. Characteristic evaluation of Trastuzumab-Erianin ADC

The ADC is then analyzed comprehensively by several methods. As shown in FIG. 4A, the reducing SDS-PAGE analysis confirms that both the light chain and heavy chain fragments of trastuzumab (mAb) migrate at a larger kDa after conjugation with erianin. The UV-Vis absorption spectrum of ADC shows the absorption peaks of both erianin and trastuzumab (FIG. 4B). By using the reversed-phase HPLC, the peaks corresponding to light chain and heavy chain fragments of the antibody shift to a longer retention time due to the conjugation of the hydrophobic molecules (FIG. 4C). Further analysis by mass spectrometry suggests that the drug-to-antibody ratio (DAR) is determined as 8, which is the theoretical maximum number of drug loading for conventional interchain cysteine conjugation (FIG. 4D). Under the native condition, no significant aggregation is observed in size-exclusion chromatogram (FIG. 4E) and the drug distribution is homogenous as confirmed by hydrophobic interaction chromatography (FIG. 4F). The binding of ADC to HER2 protein shows a dose-dependent manner with the EC₅₀ value determined to be 0.73 ± 0.03 nM by ELISA, which is not significantly different from those of mAb (0.29 ± 0.02 nM). This result indicates that the chemical modification of antibody does not significantly affect the binding affinity of the parent antibody with HER2 protein.

### Example 3. In vitro study of Trastuzumab-Erianin ADC

The selective binding and internalization behavior of ADC are then examined by flow cytometry and confocal microscopy, using two HER2-overexpressed SK-BR-3 breast cancer cells and SK-OV-3 ovarian cancer cells, as well as two HER2-negative MDA-MB-231 breast cancer cells and MCF-10A breast epithelial cells. Briefly, Different cell lines (1×10³ cells/well) are seeded in a microtiter plate (96 well) and incubated overnight before the treatment. Different concentrations of erianin, trastuzumab and ADC are added into the wells and then incubated for 6 days. After the media is removed, the cells are fixed with formaldehyde (3%; 50 µL) in PBS and stained with NBB reagent (0.05%, 0.1 M sodium acetate, 9% acetic acid; 50 µL) for overnight. The stained cells in each well are washed thrice gently with deionized water and solubilized in sodium hydroxide solution (50 mM; 100 µL). The cell viability is determined by measuring the absorbance of 620 nm in each well by microplate reader. The experiments are repeated in triplicate. The IC₅₀ values are presented as the mean ± standard deviation.

As shown in FIG. 5A and FIG. 5B, both ADC and trastuzumab specifically bind to HER2-positive cancer cells instead of HER2-negative cells at 4°C. Upon incubation at 37°C, the internalization efficiency of ADC in HER2-positive cancer cells is examined and semi-quantified by flow cytometry, showing that the internalization efficiency of the antibody and ADC is comparable and reached to 80% after 24 h (FIGs. 5C-5D). The cellular distribution of ADC is further examined by labelling with Alexa 647 (FIG. 5E). The ADC is mainly located on the cellular membrane of HER2-positive cancer cells but not on HER2-negative cell lines when incubated at 4°C. However, upon incubation at 37°C to facilitate receptor-mediated endocytosis, the ADC is appeared as red dots inside the HER2-positive cancer cells. By further staining with the LysoTracker Green (FIG. 5F), some of the red dots of ADC are located at lysosomes, indicating that the ADC can enter lysosome degradation pathway after internalizing to the HER2-positive cancer cells. The anti-proliferative activities of ADC are then investigated and compared with trastuzumab and erianin (FIGs. 5G-5I). Erianin exhibits very high cytotoxicity against all the four cell lines with IC₅₀ values ranging from 21 to 25 nM, while the cell growth inhibition of the antibody is not obvious for all the cells. In contrast, both SK-OV-3 and SK-BR-3 cancer cells with high level of HER2 expression are killed efficiently by ADC with the IC₅₀ values of 0.6 and 0.9 µM respectively but the ADC has less effects on HER2-negative cell lines (Table 1).

**Table 1. IC₅₀ values of ADC, mAb and erianin against different cell lines.**

| IC₅₀ | Trastuzumab-Erianin (ADC) | Trastuzumab (mAb) | Erianin |
|---|---|---|---|
| SK-OV-3 ovarian cancer cell (HER2+) | 0.59 ± 0.08 µM (0.088 mg/mL) | >4 µM (>0.60 mg/mL) | 21.4 ± 0.3 nM |
| SK-BR-3 breast cancer cell (HER2+) | 0.90 ± 0.07 µM (0.13 mg/mL) | >4 µM (>0.60 mg/mL) | 24.1 ± 0.1 nM |
| MDA-MB-231 breast cancer cell (HER2-) | 3.90 ± 0.05 µM (0.58 mg/mL) | >4 µM (>0.60 mg/mL) | 21.6 ± 0.2 nM |
| MCF-10A breast epithelial cell (HER2-) | >4 µM (>0.60 mg/mL) | >4 µM (>0.60 mg/mL) | 25.7 ± 0.2 nM |

### Example 3. In vivo study of Trastuzumab-Erianin ADC

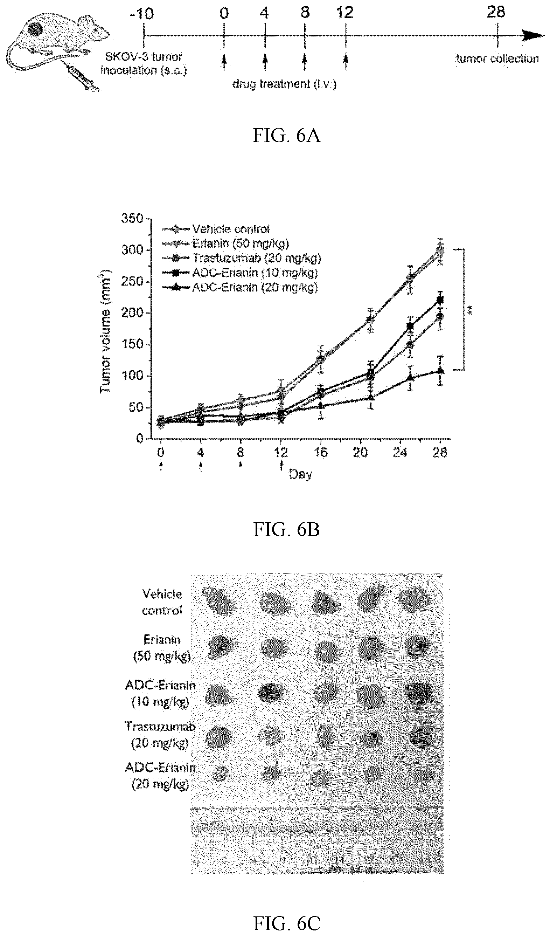

The antitumor activity of ADC is investigated in HER2-positive SK-OV-3 human ovarian cancer xenograft models (FIG. 6A). Briefly, all animal experiments are conducted under the guidelines approved by the Committee on the Use of Live Animals in Teaching and Research of the University of Hong Kong. Female BALB/cAnN-nu (nude) mice (5-8 weeks old) are housed in the Laboratory Animal Unit, The University of Hong Kong. The mice are freely access to food and water. SKOV-3 ovarian cancer cells (1 × 10⁷) in PBS (100 µL) are injected into the right flanks of each mouse subcutaneously. Once the tumor volumes reached about 50 mm³, the mice are divided into 5 groups (n = 5 for each group): (A) PBS vehicle control, (B) erianin (50 mg/kg), (C) trastuzumab (20 mg/kg), (D) ADC (10 mg/kg) and (E) ADC (20 mg/kg). The mice are treated with intravenous administration of a total of four doses on day 0, 4, 8 and 12. The tumor size and body weight are monitored during the treatment. After 28 days, the mice are sacrificed and the tumor weight is measured.

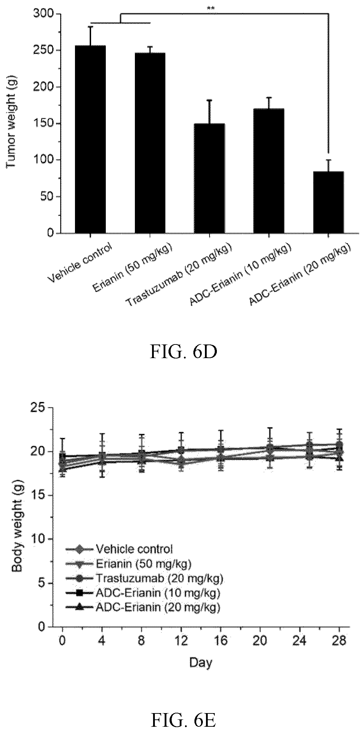

The tumor-bearing nude mice are intravenously injected with ADC (10 or 20 mg/kg) in a total of four doses for every four days. The mice are also treated with trastuzumab (20 mg/kg) and erianin (50 mg/kg) as the control. As shown in FIG. 6B, four-dose injection of erianin shows no effect on tumor growth suppression compared to vehicle control. Due to the fast metabolic clearance of erianin, high dosages and intraperitoneal injection of erianin are required and reported by other researches. While trastuzumab (20 mg/kg) shows apparent antitumor activity, treatment with ADC (20 mg/kg) significantly inhibits tumor growth with about 70% inhibition (FIG. 6C and FIG. 6D). In addition, the body weight of the mice during the treatment shows no observable change, indicating that the treatment does not exhibit obvious toxic effects (FIG. 6E).

As used herein and not otherwise defined, the terms "substantially," "substantial," "approximately" and "about" are used to describe and account for small variations. When used in conjunction with an event or circumstance, the terms can encompass instances in which the event or circumstance occurs precisely as well as instances in which the event or circumstance occurs to a close approximation. For example, when used in conjunction with a numerical value, the terms can encompass a range of variation of less than or equal to ±10% of that numerical value, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%.

Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects.

The foregoing description of the present invention has been provided for the purposes of illustration and description. It is not intended to be exhaustive or to limit the invention to the precise forms disclosed. Many modifications and variations will be apparent to the practitioner skilled in the art.

The embodiments were chosen and described in order to best explain the principles of the invention and its practical application, thereby enabling others skilled in the art to understand the invention for various embodiments and with various modifications that are suited to the particular use contemplated.

## Claims

1. An antibody-drug conjugate (ADC), comprising:
a tumor-targeting antibody; and
at least one erianin-loaded linker;
wherein the tumor-targeting antibody and the at least one erianin-loaded linker are bounded by covalent bonds;
wherein the erianin-loaded linker comprises erianin, an antibody attachment moiety, a cleavable bond and with or without a self-immolative linker.

2. The ADC of claim 1, wherein the tumor-targeting antibody comprises one or more thiol groups or one or more amino groups.

3. The ADC of claim 2, wherein the antibody attachment moiety comprises a thiol-reactive Michael acceptor group or an amine-reactive N-hydroxysuccinimide group.

4. The ADC of claim 3, wherein at least one of the thiol groups undergoes a Michael addition with the Michael acceptor group of the erianin-loaded linker to conjugate the erianin-loaded linker to the tumor-targeting antibody.

5. The ADC of claim 3, wherein the at least one of the amino groups undergoes a condensation reaction with the N-hydroxysuccinimide of the erianin-loaded linker to conjugate the erianin-loaded linker to the tumor-targeting antibody.

6. The ADC of claim 1, wherein the at least one erianin-loaded linker has the following formula: 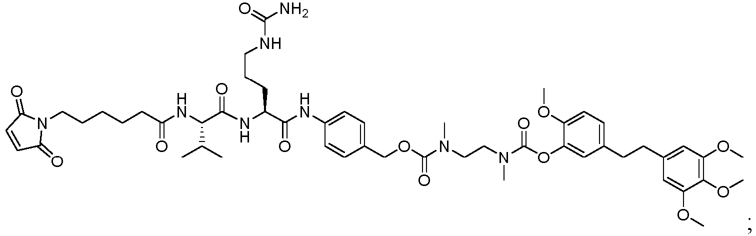 wherein the antibody attachment moiety is a maleimide moiety, the cleavable bond is a capthesin B-cleavable Val-Cit unit, and the self-immolative linker is a self-immolative p-aminobenzyl and dicarbamate linker.

7. The ADC of claim 1, wherein the ADC has 1 to 8 of the erianin-loaded linkers.

8. The ADC of claim 1, wherein the tumor-targeting antibody targets a tumor-associated receptor selected from HER2, TROP2, Nectin4, folate receptor alpha, EGFR, HER3, or c-MET.

9. The ADC of claim 1, wherein the tumor-targeting antibody is Trastuzumab.

10. A method of fabricating the ADC of claim 1, comprising:
synthesizing the erianin-loaded linker; and
conjugating the erianin-loaded linker to the tumor-targeting antibody.

11. The method of claim 10, further comprising subjecting the tumor-targeting antibody to a reducing treatment for reducing the interchain disulfide bonds to form free thiols on the tumor-targeting antibody before the conjugation.

12. A method of treating a cancer in a subject in need thereof, comprising:
administering a pharmaceutically effective amount of the ADC of claim 1 to the subj ect.

13. The method of claim 12, wherein the cancer comprises a breast cancer, lung cancer, colorectal cancer, prostate cancer, pancreatic cancer, liver cancer, esophageal cancer, gastric cancer, ovarian cancer, cervical cancer, endometrial cancer, renal cancer, bladder cancer, thyroid cancer, melanoma, non-Hodgkin lymphoma, Hodgkin lymphoma, multiple myeloma, leukemia, glioblastoma, astrocytoma, medulloblastoma, meningioma, sarcoma, bone cancer, head and neck cancer, testicular cancer, oral cancer, anal cancer, mesothelioma, neuroblastoma, retinoblastoma, and cholangiocarcinoma.

14. A pharmaceutical composition for treating cancer in a subject in need, wherein the composition comprises the ADC of claim 1 and a pharmaceutically acceptable addition, wherein the pharmaceutically acceptable addition comprises an excipient, a stability additive, a carrier, a diluent, and a solubilizer.

15. The pharmaceutical composition of claim 14, wherein the cancer comprises a breast cancer, lung cancer, colorectal cancer, prostate cancer, pancreatic cancer, liver cancer, esophageal cancer, gastric cancer, ovarian cancer, cervical cancer, endometrial cancer, renal cancer, bladder cancer, thyroid cancer, melanoma, non-Hodgkin lymphoma, Hodgkin lymphoma, multiple myeloma, leukemia, glioblastoma, astrocytoma, medulloblastoma, meningioma, sarcoma, bone cancer, head and neck cancer, testicular cancer, oral cancer, anal cancer, mesothelioma, neuroblastoma, retinoblastoma, and cholangiocarcinoma.
